Europäisches Patentamt

European Patent Office

Office européen des brevets

(11) Publication number: **0 393 973**
**A1**

(19)

## (12) EUROPEAN PATENT APPLICATION

(21) Application number: 90304085.5

(51) Int. Cl.5: **A61K 31/11, A61K 9/18**

(22) Date of filing: **17.04.90**

(30) Priority: **18.04.89 JP 97826/89**

(43) Date of publication of application:
**24.10.90 Bulletin 90/43**

(84) Designated Contracting States:
**AT BE CH DE DK ES FR GB GR IT LI LU NL SE**

(71) Applicant: **LEMON GRASS FOOD CO., LTD.**
**Miho-Bldg, 2-19, Ginza 2-chome**
**Chuo-ku, Tokyo(JP)**

(72) Inventor: **Oshiba, Sumusu**
**35-54 Koizumi, Ageo-shi**
**Saitama-ken(JP)**
Inventor: **Imai, Hideo**
**651-14, Yoshida**
**Kawagoe-shi, Saitama-ken(JP)**
Inventor: **Tamada, Terumi**
**15-15 Nishi Ikebukuro 4-chome**
**Toshima-ku, Tokyo(JP)**
Inventor: **Zenda, Hiroshi**
**4-1-2 Asahi 3-chome**
**Matsumoto-shi, Nagano-ken(JP)**
Inventor: **Hylin, John W.**
**3936 Lanipili Plaza**
**Honolulu, Hawaii(US)**

(74) Representative: **Pearce, Anthony Richmond et al**
**MARKS & CLERK Alpha Tower Suffolk Street**
**Queensway Birmingham B1 1TT(GB)**

(54) **Antitumor Agent Indicated for lung cancer.**

(57) This invention relates to an antitumor agent comprising an oral drug in which lemongrass oil is included in cyclodextrin as its effective component, and to an antitumor agent indicated for lung cancer comprising an oral drug in which citral is included in cyclodextrin as its effective component.

Lemongrass oil is a volatile liquid extracted from a natural plant, lemongrass, and citral is a volatile liquid which is a principal constituent of said lemongrass oil and which may be extracted therefrom. By suppressing their volatileness by including them in cyclodextrin, an easy-to-take oral drug without odor or bitterness which demonstrates an excellent antitumor effect specifically on lung cancer can be obtained.

## Antitumor Agent Indicated for Lung Cancer

[Industrial Field of Application]

This invention relates to an antitumor agent which acts specifically on lung cancer to demonstrate its antitumor effect.

[Prior Art]

Various attempts have heretofore been made to develop drugs effective for malignant tumors, and numerous antineoplastic agents have been introduced. Of these, major ones extensively used in chemotherapy of lung cancer are listed below.

① Alkylating agents
Cyclophosphamide (CPA)
Nimustin (ACNU)
Carboquinon (CQ)

② Metabolic antagonists
Methotrexate (MTX)
5-Fluorouracil (5-FU)
Tegaful (FT)

③ Antitumor antibiotics
Adriamycin (ADM)
Mitomycin (MMC)
Blenomycin (BLM)
Pepleomycin (PEP)

④ Vinca alkaloids
Vincristine (VCR)
Vindesine (VDS)
Epipodophyllotoxin
(Etoposide, VP-16)

⑤ Others
Cisplatin (CDDP)
Procarbazine (PCZ)

Of these antitumor agents indicated for lung cancer, CDDP developed by Rosenberg et al is used mainly for treatment of lung cancer. VP-16 and VDS are also known for their high efficacy.

[Problems To Be Solved by the Invention]

Many of the other antitumor agents, particularly antineoplastic agents indicated for lung cancer, attack cancer cells and demonstrate perfunctory effect. However, they also affect normal cells to thereby exhaust patients; its long term use is therefore not recommendable. In any case, patients rarely recover completely.

In view of the problems encountered by the conventional antitumor agents indicated for lung cancer, the inventors of this invention conducted researches for developing an antitumor agent acting specifically on lung cancer and successfully obtained a substance which is contained abundantly in and can be easily extracted from natural products and which can also be synthesized artificially for use as an effective component of said antitumor agent.

[Means To Solve the Problems]

The first of this invention is characterized in that it comprises an oral drug in which lemongrass oil obtained from natural lemongrass is included in cyclodextrin as its effective component.

The second invention is characterized in that it comprises an oral drug in which citral contained in said lemongrass oil alone is included in cyclodextrin as its effective component.

Lemongrass oil used as an effective component of the present invention antitumor agent is extracted from lemongrass grown in the tropics and utilized as a raw material for perfumes and spices.

Chemical constituents of the extract more or less differ depending on the place of origin but generally include citral, citronellol, geraniol, nerol, -terpineol, d-limonene, etc. besides numerous monoterpene alcohols and aldehydes. Said citral is contained in lemongrass oil at 70 - 80 wt%, and is expressed as $C_{10}H_{16}O$ (molecular weight: 152.24).

Said lemongrass has long been used by local residents for maintaining health by usually placing lemongrass in hot water without extraction and drinking it. However, lemongrass oil extracted from lemongrass has a unique, strong odor and pungent taste, and is not necessarily fit for drinking.

Citral, on the other hand, is a mixture of two isomers, citral-a and citral-b, and citral contained in lemongrass oil is mainly type a. It is originally a colorless or pale yellowish liquid and has a strong odor similar to lemongrass oil. It is also not quite palatable as it is.

The antitumor agent according to the first invention contains lemongrass oil extracted from such lemongrass in cyclodextrin clathrates. For cyclodextrin, a host clathrate compound, α-, β-, or γ-cyclodextrin may be used. It is preferable to use β-cyclodextrin which is relatively easily available.

Lemongrass oil obtained by steam distillation of lemongrass is added with said cyclodextrin, heated in the presence of an organic solvent, and crystallized to obtain lemongrass oil clathrates. Details of the manufacture method are described in Japanese Patent Publication Sho 63-14937 filed earlier by the present applicant.

As for the amount of lemongrass oil to be included in cyclodextrin, 5 - 15% (weight %: this applies hereinafter) of the whole lemongrass oil clathrates is recommended in view of easiness in oral administration.

If the content is less than 5%, the intake of lemongrass oil as an antitumor agent would be insufficient and therefore a larger quantity of clathrates would have to be taken. If the content exceeds 15%, on the other hand, its unique odor and pungent taste would become overwhelming.

The oral dose of such clathrates as an antitumor agent varies depending on the site of tumor, severity of symptoms, etc. and cannot be generalized. However, a small dose is enough to sustain the efficacy. Lemongrass oil clathrates thus obtained may be prepared in solid forms for oral administration such as tablets, granules, powders and capsules.

The second antitumor agent is characterized in that it is an oral drug in which only the citral contained abundantly in said lemongrass oil is included in cyclodextrin as an effective component.

In including said citral in cyclodextrin, α-, β- and γ-cyclodextrin may be used as a host compound of clathrates similarly as in the case of lemongrass oil. It is preferably to use β-cyclodextrin because of its relatively easy availability. The method disclosed in Japanese Patent Publication Sho 63-14937 may be followed similarly as for the inclusion of lemongrass oil.

The amount of citral to be included in cyclodextrin should preferably be in the range of 5 - 15% of the whole citral clathrates when considering the ease in taking the same as oral doses.

At less than 5%, the intake of lemongrass oil which acts as an antitumor agent would be insufficient, and it becomes therefore necessary to take clathrates in a larger quantity. At above 15%, the citral odor would interfere with oral administration.

The oral dose of such clathrates as an antitumor agent varies depending on the tumor site, severity of symptoms, etc. and cannot be generalized. However, a small dose would be enough to sustain the efficacy. These clathrates may be prepared in solid forms for oral administration such as tablets, granules, powders and capsules similarly as in the case of lemongrass oil.


[Action]


A principal constituent of lemongrass oil is citral, which is contained at 70 - 80%.

Boyland et al conducted a study by oral administration of citral to mice bearing sarcoma 180 and reported that this compound suppresses growth of cancer cells. (Boyland, E. and Mawson, E.H.: Experiments on the chemotherapy of cancer II. The effect of aldehydes and glucosides. Chemotherapy of Cancer, 1982-1987, 1938)

On the other hand, citral is reported to have a hemolytic activity (Tamir, I., Abramovici, A., Milo-Goldzweig, I. and Segal, R.: The hemolytic activity of citral; evidence for free radical partication. Biochem. Pharmacology, 19, 2945-2950, 1984) and to be teratogenic to chicken embryos (Abramovici, A. and Richmuth-Roizman, P.: Molecular structure teratogenicity relationships of some fragrance additives. Toxicology, 29, 143-156, 1983).

Based on these references on citral, the present inventors carried out various tests and obtained the following results.

(1) When lemongrass oil of a high concentration or citral was directly given to normal rats orally by gavage, petechiae were observed on the gastric mucosa. It was therefore assumed that a massive dose of citral or lemongrass oil which contains citral as its principal constituent would adversely affect normal cells.

(2) Instead of dosing lemongrass oil directly to normal rats, lemongrass oil or citral in $\beta$-cyclodextrin clathrates was mixed with feed. No abnormalities were observed in the gastric mucosa, etc. of the animals. $\beta$-cyclodextrin which is a derivative of starch was presumably decomposed by amylase in pancreatic juice as said clathrates passed through the digestive tract of mice together with diet, and was gradually released to allow lemongrass oil or citral to be absorbed in the small intestine to thereby decrease damages to the intestinal mucosa.

(3) When said lemongrass oil or citral clathrates were given to mice carrying cancer of the liver, the effect to supress cancer growth was hardly noted. Unexpectedly, however, there was no metastasis to the lung.

As is evident from the latter experiment, there were clearly significant differences between the cancer bearing mice orally given lemongrass oil clathrates and those treated with no lemongrass oil or citral in the number of lung metastases, the rate of non-metastasis to the lung and the survival time, indicating excellent effects of lemongrass in suppressing metastasis and extending survival.

The effect of lemongrass oil or citral clathrates on lung cancer, namely suppression of lung metastasis and extension of survival time, was attributed to the following specific actions.

(a) An action of a single or composite component of lemongrass oil to work directly on the lung and attack the cancer cells metastasized to the lung.

(b) Lemongrass oil or citral per se strongly suppresses platelet aggregation. An action to prevent metastasis of cancer cells by this suppression of platelet aggregation.

(c) As lemongrass oil and citral are highly volatile, they are partially excreted from blood into the expired air after absorption. An action of lemongrass or citral to contact the metastatic site in the lung in gaseous form to thereby suppress cancer cell growth.

From the results described in (1) - (3), it was concluded that lemongrass oil or citral clathrates in cyclodextrin are effective as an antitumor agent for lung cancer and that an intensely effective application may be achieved by preparing them into oral doses.

Acute toxicity studies on these lemongrass oil and citral clathrates revealed them to be quite safe as shown below.

| Lemongrass oil | |
| --- | --- |
| Dose (mg/kg) | Deaths/number of subjects |
| 2,000 | 2/10 |
| 1,000 | 0/10 |
| Citral | |
| Dose (mg/kg) | Deaths/number of subjects |
| 2,000 | 3/10 |
| 1,000 | 0/10 |

In the above studies, healthy, male dd strain mice weighing 28 - 33 g were used. Lemongrass oil and citral were respectively suspended in 10% acacia solution and orally administered by gavage at 2,000 mg/kg and 1,000 mg/kg to ten mice/level/compound. Survival of mice was examined one week later.

Brief Description of the Drawings

Fig. 1 shows the time table for administration of mouse feeds added with lemongrass oil clathrates; (a) for Experiment I and (b) for Experiment II. Fig. 2 shows chronological changes in body weight of mice in Experiment I and Fig. 3 those in Experiment II.

Fig. 4 shows the rates of metastasis and non-metastasis to the lung in cancer bearing mice in Experiment II, Fig. 5 viability of mice in Experiment I and Fig. 6 that of mice in Experiment II.

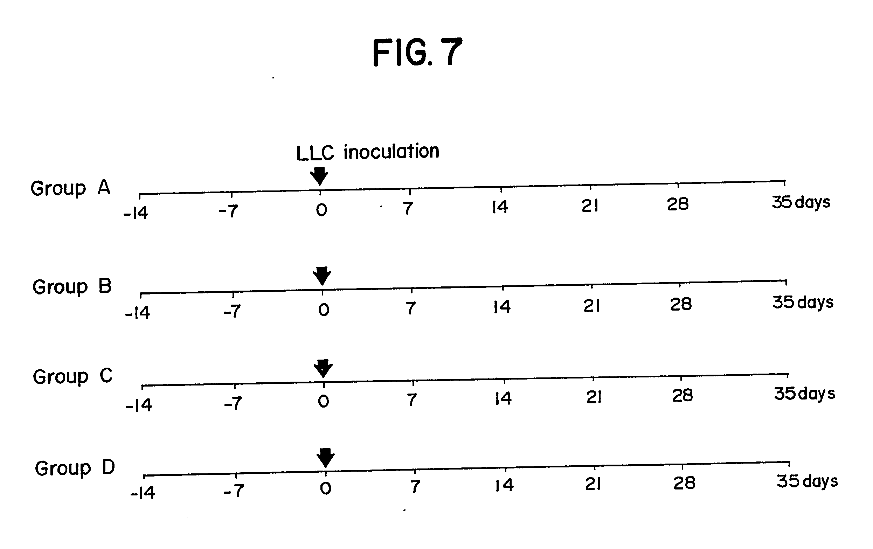

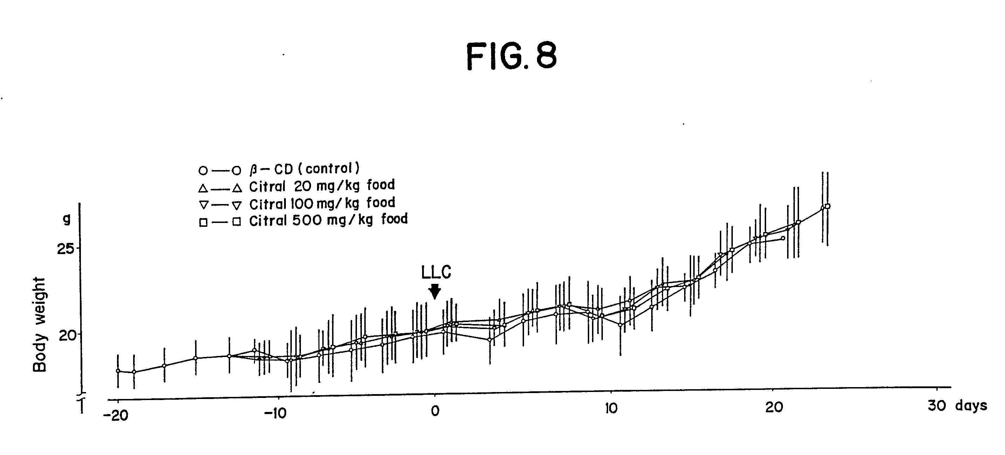

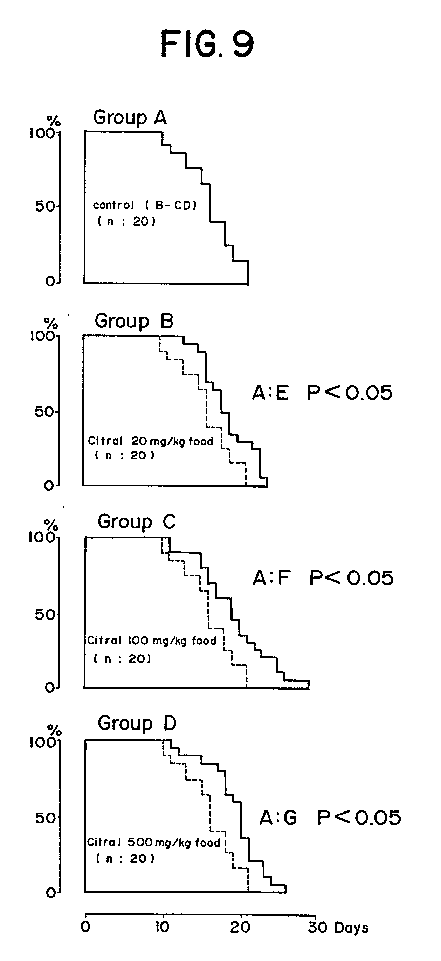

To explain the experiment on citral clathrates, Fig. 7 shows the time table for administration of test feeds to mice, Fig. 8 chronological changes in body weight of these mice and Fig. 9 their viability.

[Examples]

The pharmacological action of the present invention antitumor agent and its effects will be explained in detail by way of examples.

Experiment on lemongrass oil clathrates

The present invention antitumor agent was tested as follows by administration to mice.

(1) Preparation of feed

One ml of lemongrass oil (hereinafter "LGO") was dissolved in 50 ml of isopropyl alcohol and added with 50 ml of distilled water and 15 g of $\beta$-cyclodextrin (hereinafter "$\beta$-CD"). The resultant mixture was placed in a flask, heated to 75 - 80°C in a water bath for one hour while stirring, and allowed to cool spontaneously to obtain clathrates. Gas chromatographic analysis revealed 70 - 80 mg of LGO in 1 g of clathrates and its components unchanged.

Untreated mouse feed for controls (CE-2, Clea Japan, Inc.; hereinafter "control feed") was prepared in addition to three types of feeds containing LGO at ① 20 mg, ② 100 mg and ③ 1,000 mg per 1 kg.

(2) Test animals and formation of test tumor cells

Male C57BL/6N mice (Charles River, Japan) weighing 16.9 ± 0.9 g were used as test animals. Lewis lung carcinoma (hereinafter abbreviated as "LLC") obtained by subinoculation of spontaneous lung cancer of the mice was implanted at an implantation site to form a tumor. This tumor mass was sterilely extirpated, cut into thin sections and processed routinely. Live cells were then counted and immediately inoculated subcutaneously at the femoral region of mice at $2 \times 10^5$ cells/0.2 ml. Subsequently, presence or absence of visible lung metastases and other developments were examined.

(3) Method

The experiment was performed in two phases, Experiment I and Experiment II.

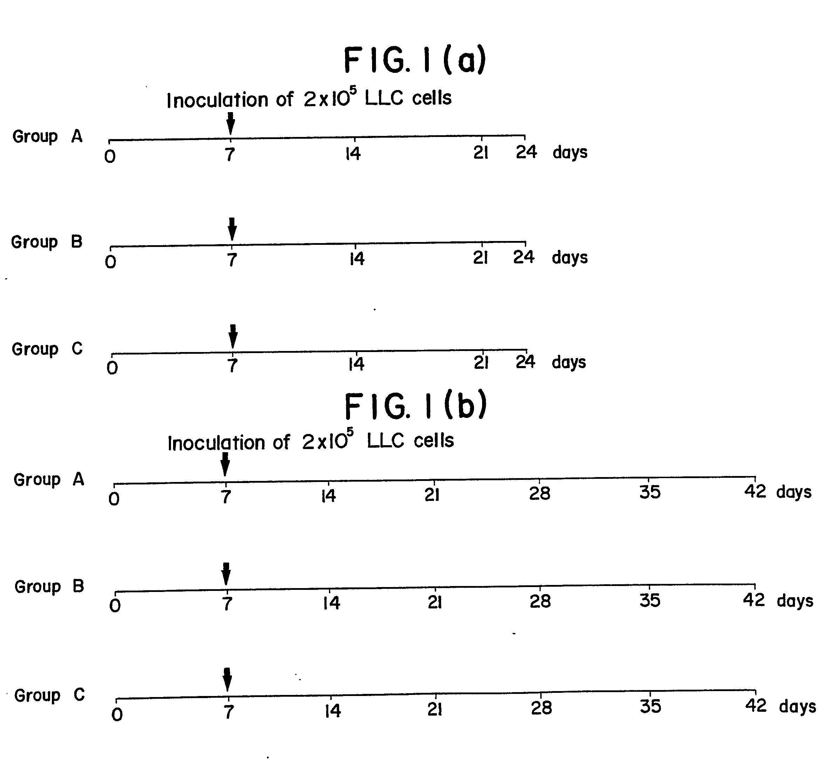

Three groups were used in Experiment I as shown in Fig. 1 (a); Group A comprising 10 control mice given the control feed, Group B comprising 16 mice given the feed containing LGO at 20 mg/kg, and Group C comprising 16 mice given the feed containing LGO at 100 mg/kg. Animals were given the respective feeds one week prior to LLC inoculation.

In Experiment II, three groups were used as shown in Fig. 1 (b); Group A comprising 40 control mice, Group B comprising 40 mice given the feed containing LGO at 100 mg/kg, and Group C comprising 40 mice given the feed containing LGO at 1,000 mg/kg. Animals were given the respective feeds from one week prior to LLC inoculation.

The measurement items are as follows.

① Food consumption and body weight

Food consumption and body weight were measured once every two days.

② Weight of the tumor mass at the implantation site

After sacrifice, the femoral region of mice was cut out and fixed in neutral formalin for 48 hours. The skin, fat and bone were then removed to determine the weight of the tumor mass.

③ Observation of lung metastases

Mice were sacrificed by cervical dislocation, and diluted Indian ink (15 ml of Pelikan 17 black, W. Germany + 1 ml of ammonia water + 85 ml of distilled water) was infused from the trachea into the lung according to Wexler's method. Following extirpation, the lung was immediately fixed in neutral isotonic formalin.

Metastases were recognized as white spots or nodes on the lung surface dyed by said diluted Indian ink, and counted under a stereoscopic microscope.

④ Histological observation of the implantation site and the lung

The tumor mass at the implantation site and the lung were fixed in neutral isotonic formalin, embedded in paraffin, cut into thin sections and stained with hematoxylin-eosin or Giemsa for histological observation under a light microscope.

(4) Results

① Food consumption

The mean food consumption/mouse/day in Experiment I was 3.2 ± 0.5 g in Group A, 3.3 ± 0.4 g in Group B and 3.2 ± 0.6 g in Group C, indicating no intergroup differences.

The mean food consumption/mouse/day in Experiment II was 3.1 ± 0.4 g in Group A, 3.1 ± 0.3 g in Group B and 3.1 ± 0.3 g in Group C, indicating no intergroup differences.

The LGO intake/mouse/day calculated from the food consumption was about 0.06 mg (about 2.4 mg/l kg body weight) in Group B and about 0.3 mg (about 12 mg/l kg body weight) in Group C in Experiment I, and about 0.3 mg (about 12 mg/l kg body weight) in Group B and about 3 mg (about 120 mg/l kg body weight) in Group C in Experiment II.

② Body weight changes in cancer bearing mice

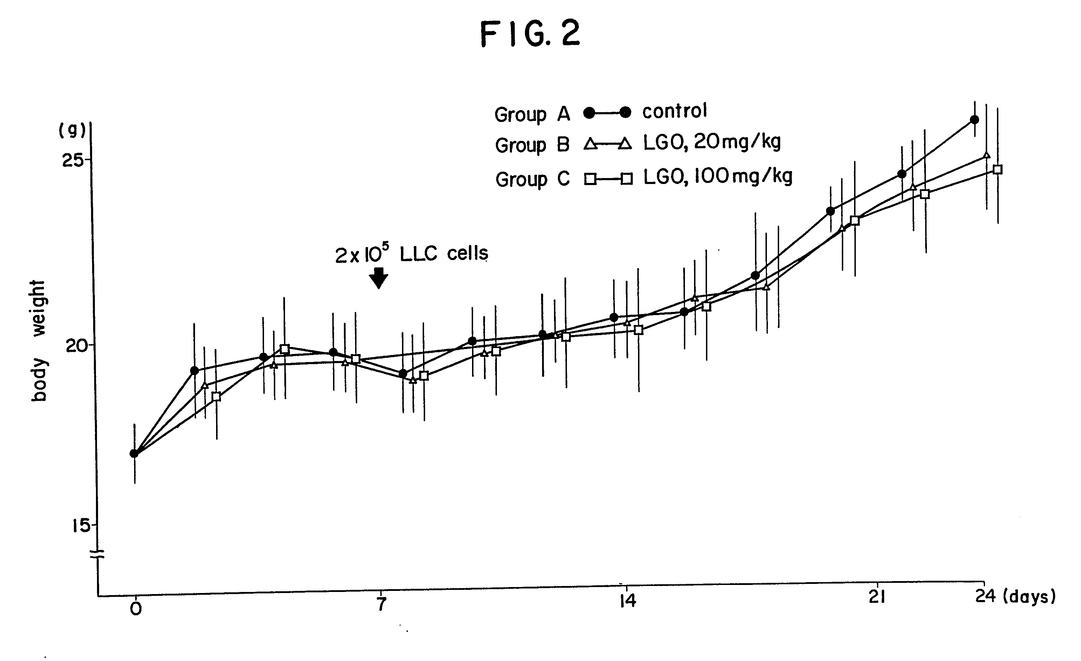

Changes in body weight of mice in Experiment I are shown in Fig. 2. As shown in the figure, although body weights of Groups A, B and C somewhat decreased transiently immediately after LLC inoculation, comparable body weight gain was subsequently observed in all the three groups.

A thumb-sized tumor was formed by Day 17 after LLC inoculation. There was no intergroup difference in respect of the time of tumor formation at the implantation site.

A marked body weight gain observed after Day 17 after LLC inoculation was attributed to a rapid growth of the tumor at the implantation site. No intergroup differences were observed in the weight of the tumor mass.

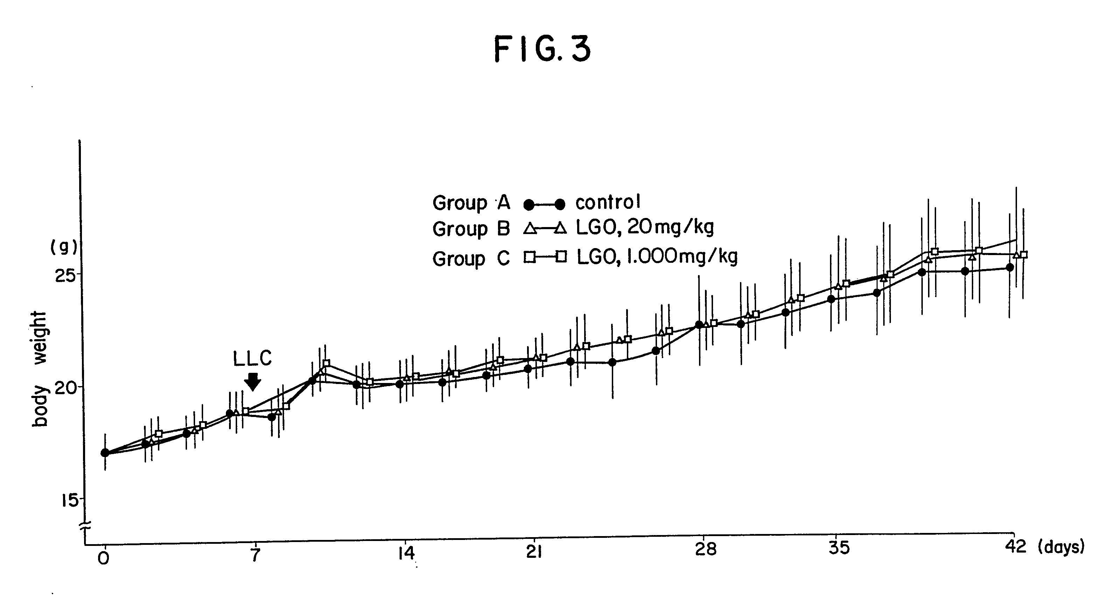

As shown in Fig. 3, body weight gains in Experiment II were comparable among Groups A, B and C. In all these groups, a visible tumor mass was formed at the implantation site after Day 20 after LLC inoculation, which grew into a thumb-sized tumor by Day 35. No intergroup differences were observed in respect of the time of tumor formation at the inoculation site nor any difference in the weight of the tumor mass.

③ Number of lung metastases

Dispersed LLC cells develop into a tumor at the inoculation site and then metastasize to the lung. In Experiment I, the number of lung metastases was 36 ± 2 in Group A, 22 ± 9 in Group B and 27 ± 11 in Group C, indicating that lung metastases were fewer in Group B than in A (p < 0.05). The number of metastases in Group C was also fewer than in A in terms of the mean value. Light microscopic observations revealed no intergroup differences in the histological picture of the lung metastases.

In Experiment II, on the other hand, the number of lung metastases was 22 ± 8 in Group A, 13 ± 6 in Group B and 17 ± 12 in Group C, indicating that metastases were markedly fewer in Group B than in A (p < 0.05) and that they were also fewer in Group C than in A in terms of the mean value. Similarly as in Experiment I, light microscopic observations of the lung metastases revealed no intergroup differences in their histological picture.

④ Rate of non-metastasis to the lung

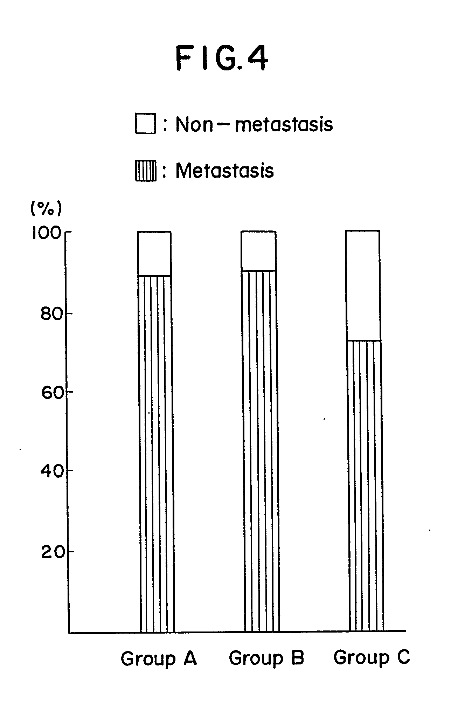

Metastasis was not observed in some animals in Experiment II. As shown in Fig. 4, the rate of non-metastasis to the lung in mice inoculated with LLC was 5/40 (12%) in Group A, 4/40 (10%) in Group B and 13/40 (32%) in Group C. A marked suppression of lung metastasis was observed in Group C which was given LGO of a high concentration.

⑤ Effect of LGO on survival time

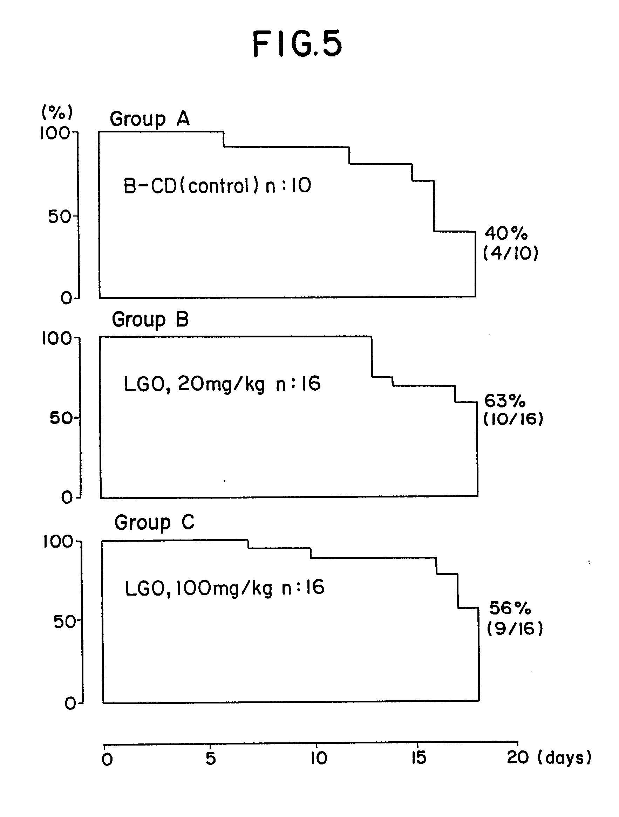

The survival time of cancer bearing mice in Experiment I is presented in Fig. 5. Viability on Day 17 after LLC inoculation was 40% (4/10 were alive) for Group A, 63% (10/16 alive) for Group B and 56% (9/16 alive) for Group C. Viabilities in the LGO groups, B and C, were evidently higher than in Group A given no LGO although there was no difference between B and C.

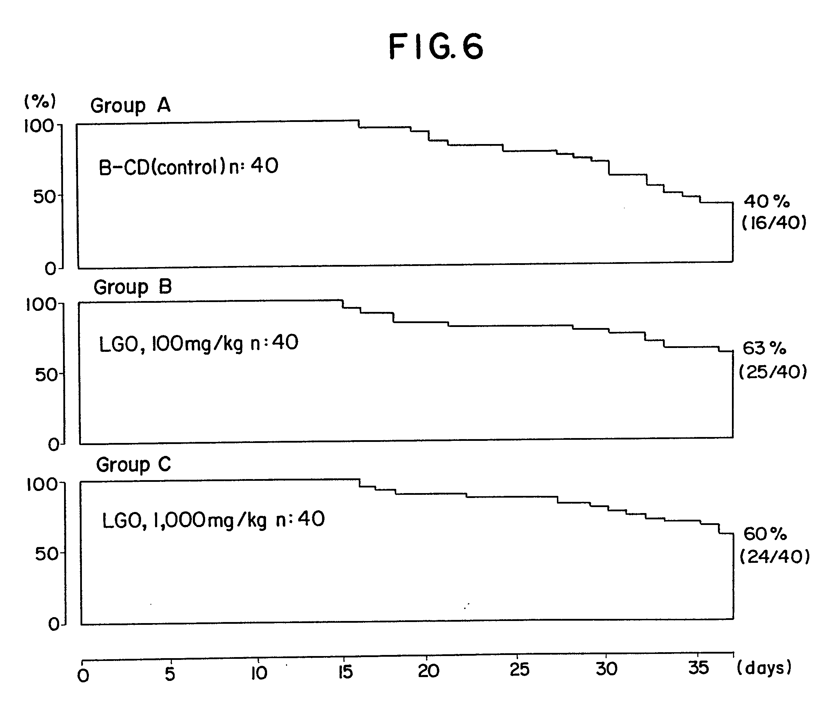

The survival time of cancer bearing mice in Experiment II is presented in Fig. 6. Viability on Day 35 after LLC inoculation was 40% (16/40 alive) for Group A, 63% (25/40 alive) for Group B and 60% (24/40 alive) for Group C, showing that the viabilities in the LGO groups, B and C, were as high as 1.5 times that of Group A given no LGO.

Experiment on citral clathrates

(1) Preparation of feed

One ml of citral extracted routinely from lemongrass oil was dissolved in 50 ml of isopropyl alcohol and added with 50 ml of distilled water and 15 g of $\beta$-cyclodextrin (hereinafter "$\beta$-CD"). The mixture was placed in a flask, heated for one hour to 75 - 80°C in a water bath while stirring, and allowed to cool spontaneously to obtain clathrates. Gas chromatographic analysis revealed 70 - 80 mg of citral in 1 g of the clathrates and its components unchanged.

Untreated mouse feed was prepared for control animals (CE-2, Clea Japan, Inc.; hereinafter "control feed") in addition to feeds containing ① 20 mg, ② 100 mg and ③ 500 mg of said clathrates in 1 kg of said mouse feed.

(2) Test animals and formation of test tumor cells

Male C57BL/6N mice (Charles River, Japan) weighing 16.2 - 17.5 g and Lewis lung carcinoma (hereinafter abbreviated as "LLC") cells obtained by subinoculation of spontaneous lung cancer of these mice were used for the study. LLC was implanted at an implantation site to form a tumor. The tumor mass was sterilely extirpated, cut into thin sections and routinely processed. Live cells were then counted, immediately inoculated subcutaneously at the femoral region of mice at $2 \times 10^5$ cells/0.2 ml and observed of their subsequent growth.

(3) Method

As shown in Fig. 7, four groups were used in the test; i.e. Group A comprising 20 control mice given the control feed, Group B comprising 20 mice given the feed containing citral at 20 mg/kg, Group C comprising 20 mice given the feed containing citral at 100 mg/kg and Group D comprising 20 mice given the feed containing citral at 500 mg/kg. Animals were given the respective feeds from two weeks prior to LLC inoculation. The following measurement and observations were made.

① Body weight

Mice were weighed once every two days between 9:00 and 10:00 a.m.

② Observation of survival and death

Mice were daily observed for their growth conditions at 9:00 a.m., 1:00 and 6:00 p.m., and deaths were counted and recorded on each occasion.

(4) Results

① Citral intake

The citral intake/mouse/day calculated from food consumption was about 0.06 mg (about 2.4 mg/l kg body weight) in Group B given the feed containing citral at 20 mg/kg, about 0.3 mg (about 12 mg/l kg body weight) in Group C given the feed containing citral at 100 mg/kg, and about 1.5 mg (about 80 mg/l kg body weight) in Group D given the feed containing citral at 500 mg/kg.

② Body weight changes of mice and tumor formation

As shown in Fig. 8, body weights somewhat decreased transiently on Day 3 after LLC inoculation in Groups A, B, C and D. Subsequently, similar body weight gains were observed in all the groups from Day 10 to Day 12. Around this time, a visible tumor mass developed at the site inoculated with LLC in all the groups, which grew into a thumb-sized tumor after Day 16. There were no intergroup differences in the time of tumor formation at the implantation site or in the time of its growth.

③ Effect of citral on survival time of LLC-implanted mice

Fig. 9 shows the survival time of cancer bearing mice. Review on the survival of these mice throughout the test period revealed a significant ($p < 0.05$) macrobiotic effect in Groups B, C and D as compared to Group A, while no significant differences were observed in the survival time among Groups B, C and D. In Fig. 9, the survival time of Group A is shown by dotted lines in graphs for B, C and D.

Table 1 shows the mean survival time (MST) of each group and the rate of increase (%) in MST against Group A in the remaining groups. A macrobiotic effect is apparent in Groups B - D.

Table 1

|  | MST (days) | Rate of increase in MST against Group A (%) |
|---|---|---|
| Group A | 15.7 |  |
| Group B | 18.1 | 15 |
| Group C | 19.3 | 23 |
| Group D | 20.5 | 31 |

[Effect of the Invention]

The antitumor agent according to the present invention comprises an oral drug in which lemongrass oil obtained from lemongrass or citral contained in lemongrass oil is included in cyclodextrin as its effective component. It acts specificially on lung cancer, suppresses formation of lung metastases and effectively extends survival.

The present invention antitumor agent is excellent in that, by being included in cyclodextrin, lemongrass oil or citral either of which has a strong odor and pungent taste is made into an easy-to-take oral drug which demonstrates an antitumor effect intensely on lung cancer without damaging the digestive system.

Lemongrass oil which constitutes an effective component of this antitumor agent is originally a natural plant extract, and citral can be easily extracted from said lemongrass oil. Cyclodextrin which is used for inclusion of either of these is also non-toxic and widely used in various food applications, indicating it is free from toxicity.

## Claims

1. An antitumor agent for the treatment of lung cancer, which is for oral administration and which contains lemongrass oil included in a cyclodextrin clathrate.

2. An antitumor agent as claimed in Claim 1, wherein said lemongrass oil is included in the cyclodextrin clathrate in an amount of 5 - 15 wt% of the total weight of the clathrate.

3. An antitumor agent for the treatment of lung cancer, which is for oral administration and which contains citral substantially free of other components contained in lemongrass oil, included in a cyclodextrin clathrate.

4. A antitumor agent as claimed in Claim 3, wherein said citral is included in cyclodextrin in an amount of 5 - 15 wt% of the total weight of the clathrate.

5. An antitumor agent as claimed in Claim 3 or 4, wherein said citral is that which has been manufactured by catalytic air oxidation of geraniol or nerol.

6. An antitumor agent as claimed in Claim 3 or 4, wherein said citral is that which has been isolated from lemongrass oil.

7. The use of a cyclodextrin clathrate including lemongrass oil or citral in the manufacture of an antitumor agent for the treatment of lung cancer by oral administration.

· Claims for the following Contracting States: ES, GR

1. A method of manufacturing an antitumor agent for the treament of lung cancer by oral administration, comprising the steps of extracting lemongrass oil from lemongrass, and forming a cyclodextrin clathrate including said lemongrass oil.

2. A method as claimed in claim 1, wherein the lemongrass oil is included in the clathrate in an amount of 5 - 15% by weight based on the total weight of the clathrate.

3. A method of manufacturing an antitumor agent for the treatment of lung cancer by oral administration, comprising the steps of providing citral which is substantially free of other lemongrass oil components, and forming a cyclodextrin clathrate including said citral.

4. A method as claimed in claim 4, wherein said citral is included in the clathrate in an amount of 5 -15% by weight of the total weight of the clathrate.

5. A method as claimed in claim 3 or 4, wherein the citral is provided by catalytic oxidation of geraniol or nerol.

6. A method as claimed in claim 3 or 4, wherein the citral is provided by isolating it from lemongrass oil.

7. The use of a cyclodextrin clathrate including lemongrass oil or citral in the manufacture of an antitumor agent for the treatment of lung cancer by oral administration.

# FIG. I (a)

Inoculation of $2 \times 10^5$ LLC cells

**Group A**

| 0 | 7 | 14 | 21 | 24 days |

**Group B**

| 0 | 7 | 14 | 21 | 24 days |

**Group C**

| 0 | 7 | 14 | 21 | 24 days |

# FIG. I (b)

Inoculation of $2 \times 10^5$ LLC cells

**Group A**

| 0 | 7 | 14 | 21 | 28 | 35 | 42 days |

**Group B**

| 0 | 7 | 14 | 21 | 28 | 35 | 42 days |

**Group C**

| 0 | 7 | 14 | 21 | 28 | 35 | 42 days |

EP 0 393 973 A1

FIG. 2

# FIG.3

Group A ●—● control
Group B △—△ LGO, 20mg/kg
Group C □—□ LGO, 1.000mg/kg

LLC

body weight (g)

25

20

15

0       7       14      21      28      35      42 (days)

EP 0 393 973 A1

# F I G.4

☐ : Non—metastasis

▦ : Metastasis

EP 0 393 973 A1

# FIG.5

(%)

**Group A**

100
B–CD(control) n:10

50

40%
(4/10)

0

**Group B**

100
LGO, 20mg/kg n:16

50

63%
(10/16)

0

**Group C**

100
LGO, 100mg/kg n:16

50

56%
(9/16)

0

0    5    10    15    20 (days)

FIG.6

# FIG. 7

LLC inoculation

Group A
-14    -7    0    7    14    21    28    35 days

Group B
-14    -7    0    7    14    21    28    35 days

Group C
-14    -7    0    7    14    21    28    35 days

Group D
-14    -7    0    7    14    21    28    35 days

EP 0 393 973 A1

# FIG. 8

Legend:
- ○—○ β − CD ( control )
- △—△ Citral 20 mg/kg food
- ▽—▽ Citral 100 mg/kg food
- □—□ Citral 500 mg/kg food

LLC

Body weight (g)

25

20

-20   -10   0   10   20   30 days

EP 0 393 973 A1

# FIG. 9

Group A

%

100

50

0

control ( B-CD)
( n : 20 )

Group B

%

100

50

0

A:E  P< 0.05

Citral  20 mg/kg food
( n : 20 )

Group C

%

100

50

0

A:F  P< 0.05

Citral 100 mg/kg food
( n : 20 )

Group D

%

100

50

0

A:G  P< 0.05

Citral 500 mg/kg food
( n : 20 )

0        10        20        30 Days

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-3318 (CHIMICASA)<br>* page 8, lines 24 - 27 *<br>--- | 1-7 | A61K31/11<br>A61K9/18 |
| A | WO-A-8808304 (CHINOIN GYOGYSZER ES VEGYESZETI TERMEKEK GYARA RT)<br>* the whole document *<br>--- | 1-7 | |
| A,D | PATENT ABSTRACTS OF JAPAN<br>vol. 10, no. 335 (C-384) 13 November 1986,<br>& JP-A-61 139360 (REMON GLASS FOOD)<br>* the whole document *<br>--- | 1-7 | |
| A | PATENT ABSTRACTS OF JAPAN<br>vol. 4, no. 73 (C-12)(555) 28 May 1080,<br>& JP-A-55 038338 (RIKAGAKU KENKYUSHO) 17 March 1980,<br>* the whole document *<br>--- | 1-7 | |
| A | PATENT ABSTRACTS OF JAPAN<br>vol. 11, no. 18 (C-398)(2465) 17 January 1987,<br>& JP-A-61 192263 (REMON GURASUFUUDE) 26 August 1986,<br>* the whole document *<br>----- | 1-7 | TECHNICAL FIELDS SEARCHED (Int. Cl.5 )<br><br>A61K |

The present search report has been drawn up for all claims

| Place of search | Date of completion of the search | Examiner |
|---|---|---|
| BERLIN | 16 JULY 1990 | AVEDIKIAN P.F. |